# EUROPEAN PATENT APPLICATION

(11) **EP 4 353 161 A1**
(43) Date of publication of application: **17.04.2024**
(21) Application number: 22836784.3
(22) Date of filing: 29.06.2022
(51) Int. Cl.: A61B 17/22, A61B 17/221

(54) **STENT AND THROMBECTOMY SYSTEM**

(30) Priority: 09.07.2021 CN 202110778182
(71) Applicant: MicroPort NeuroTech (Shanghai) Co., Ltd., Shanghai 201318 (CN)
(72) Inventor: LIU, Jianmin, Shanghai 201318 (CN); HU, Xintong, Shanghai 201318 (CN); MENG, Fanhe, Shanghai 201318 (CN); TIAN, Hao, Shanghai 201318 (CN); HOU, Juan, Shanghai 201318 (CN)
(74) Representative: Marks & Clerk LLP
(86) International application number: PCT/CN2022/102242
(87) International publication number: WO 2023/280030

(57) **Abstract**

Disclosed are a stent and a thrombectomy system including the stent. The stent includes a stent body (4) and a radiopaque structure, the stent body formed by coupling at least one closed-loop net unit (400, 401) with each other, the closed-loop net unit (400, 401) each enclosed by a strut, the radiopaque structure including a main radiopaque member (2) having at least a main radiopaque wire (201, 202, 203), and the main radiopaque wire (201, 202, 203) wound around at least part of the stent body (4) along the strut. The thrombectomy system includes the stent described above and a push rod (5) provided at a proximal end of the stent. The described arrangement makes it possible to clarify positions of a first closed-loop net unit (400) and a second closed-loop net unit (401) on the stent body (4), thereby facilitating a surgeon determining the position of the stent body (4), providing more information for the surgeon to determine the position of the stent, and further improving visibility of the stent body (4).

## Description

### TECHNICAL FIELD

The present invention relates to the technical field of medical devices, and in particular to a stent and a thrombectomy system including the stent.

### BACKGROUND

Stroke, also known as cerebral apoplexy, is an acute cerebrovascular disease with symptoms of cerebral ischemia and hemorrhagic damage as its main clinical manifestations. The "China Stroke Prevention and Treatment Report" released in 2018 pointed out that stroke in our country presents five characteristics: high incidence, high disability rate, high mortality, high recurrence rate, and high economic burden. Stroke has become the number one cause of death in our country. On average, one person has a stroke every 12 seconds, and one person dies of a stroke every 21 seconds. According to the comprehensive standardized prevalence rate in 2016, as many as 1.96 million patients died of stroke nationwide every year, and 70% of survivors had varying degrees of permanent disability. Acute ischemic stroke accounts for about 70% to 80% of all stroke patients. The key to treatment is to restore cerebral blood flow as soon as possible and reduce secondary brain damage before permanent damage to brain tissue due to ischemia occurs. Currently, there are two main categories of treatments for acute ischemic stroke: drug thrombolysis and mechanical thrombectomy.

Intra-arterial drug thrombolysis and intravenous drug thrombolysis are conventional methods for the treatment of acute ischemic stroke. Although it has been shown that drug thrombolysis is able to improve the prognosis of the nervous system, it still faces some problems. First, the time window for thrombolysis is short. Intravenous thrombolysis should be performed within 3 hours after the onset of illness. The time window for intra-arterial thrombolysis is only 6 hours. The extremely short time window results in that only few patients can receive thrombolytic treatment; secondly, the vascular recanalization time of drug thrombolysis is long, and the vascular recanalization time may be an important factor affecting clinical prognosis, while the vascular recanalization times of intra-arterial thrombolysis and intravenous thrombolysis are at least 1 to 2 hours; thirdly, thrombolytic treatment is only suitable for patients with small volume thrombus, and the recanalization rate of acute ischemic stroke caused by severe large vessel occlusion is low; finally, some patients are not suitable for thrombolytic treatment.

In order to solve the above-mentioned problems in drug thrombolysis, the use of mechanical methods to eliminate thrombus has become a hot research topic in recent years. Intra-arterial mechanical thrombectomy devices have received widespread attention because of their many advantages: rapid vessel recanalization, longer stroke intervention time window, lower bleeding rate, and satisfactory clinical results in recanalization especially for acute ischemic stroke caused by large vessel occlusion.

At present, existing mechanical thrombectomy usually uses intracranial thrombectomy devices. Intracranial thrombectomy devices are usually metal stents made by laser cutting processing technology. However, these metal stents still have many problems to be solved. One of the main problems is that the group of radiopaque points or radiopaque wires on the metal stent can only show the positioning or outline of the metal stent on X-ray during surgery. Not all the metal rods of the laser-cut metal stent can be shown. The doctor cannot determine the release and local expansion of the main body of the thrombectomy device on X-ray, but can determine whether thrombus is caught only after the thrombectomy device is removed from the human body. This is not conducive to the doctor's judgment during the operation and wastes valuable treatment time.

In order to improve the problem in visibility, a thrombectomy device for blood vessels has also appeared on the market. In addition to multiple radiopaque points at the distal end of the stent, this thrombectomy device also has multiple groups of mark points arranged in different portions of the main body of the stent. Doctor can use the mark points to determine the position and direction of the thrombectomy device within the blood vessel. However, the visibility of the above-mentioned thrombectomy device is far from optimal.

### SUMMARY OF THE INVENTION

In view of the above defects in the prior art, an object of the present invention is to provide a stent and a thrombectomy system including the stent to solve one or more problems in the prior art.

In order to achieve the above objects, the technical solutions of the present invention are as follows.

A stent, comprising a stent body and a radiopaque structure, the stent body formed by coupling at least one closed-loop net unit with each other, the closed-loop net unit each enclosed by struts, the radiopaque structure comprising a main radiopaque member having at least a main radiopaque wire wound around at least part of the stent body along the struts.

Further, the main radiopaque wire is spirally wound around the struts.

Further, the main radiopaque wire is arranged from a proximal end to a distal end of the stent body or from a distal end to a proximal end of the stent body.

Further, the main radiopaque wire forms a radiopaque net unit in a path in which the strut is wound around the stent body, and the radiopaque net unit is configured to indicate a structure and/or position of the stent.

Further, the radiopaque net unit has a closed geometric shape; or the radiopaque net unit has a semi-open geometric shape.

Further, an area of the radiopaque net unit is same as an area of the closed-loop net unit of the stent body at a position where the radiopaque net unit is; or
an area of the radiopaque net unit is smaller than an area of the closed-loop net unit of the stent body at a position where the radiopaque net unit is; or
an area of the radiopaque net unit is a sum of areas of a plurality of the at least one closed-loop net unit of the stent body at a position where the radiopaque net unit is.

Further, the closed-loop net unit each comprises at least a first closed-loop net unit and at least a second closed-loop net unit, and an area enclosed by the first closed-loop net unit is larger than an area enclosed by the second closed-loop net unit.

Further, the area enclosed by the first closed-loop net unit is 2 to 5 times the area enclosed by the second closed-loop net unit.

Further, a number of the first closed-loop net units is 1/20-1/3 of a number of the second closed-loop net units.

Further, the main radiopaque wire is wound onto at least one of the first closed-loop net units, so that the at least one of the first closed-loop net units of the stent body can be indicated.

Further, the main radiopaque wire is wound around all the struts of the at least one of the first closed-loop net units and wound around a part of the struts of at least one of the second closed-loop net units.

Further, a part of the struts of at least one of the first closed-loop net units is wound at least twice by the main radiopaque wire.

Further, the main radiopaque wire is wound around a part of the struts of the at least one of the first closed-loop net units and wound around a part of the struts of at least one of the second closed-loop net units.

Further, a number of the main radiopaque wires is at least two, and wherein one of the main radiopaque wires is wound around a part of the struts of the at least one of the first closed-loop net units and wound around a part of the struts of at least one of the second closed-loop net units, and another one of the main radiopaque wires is wound around a rest of the struts of the at least one of the first closed-loop net units and wound around a part of the struts of at least one of the second closed-loop net units.

Further, a number of the main radiopaque wires ranges from 1 to 8.

Further, the radiopaque structure further comprises a distal radiopaque member connected to a distal end of the stent body.

Further, a proximal end of the distal radiopaque member is integrally or detachably connected to a distal end of the main radiopaque member.

Further, the distal radiopaque member is a first distal radiopaque wire that is wound around a distal strut of the stent body to form at least one layer of wrapping structure.

Further, the distal radiopaque member is a second distal radiopaque wire that is wound around a distal strut of the stent body to form an axial ring and further wound around an outer side of the axial ring to form a circumferential ring.

Further, the distal radiopaque member is arranged coaxially or non-coaxially with the stent body.

Further, the radiopaque structure further comprises a proximal radiopaque member connected to a proximal end of the stent body.

Further, a distal end of the proximal radiopaque member is integrally or detachably connected to a proximal end of the main radiopaque member.

Further, the proximal radiopaque member is arranged coaxially or non-coaxially with the stent body.

The present invention also provides a thrombectomy system, comprising the above stent and a push wire provided at a proximal end of the stent, the push wire arranged coaxially or non-coaxially with the stent.

Compared with the prior art, the beneficial technical effects of the present invention are as follows:
(1) The stent of the present invention includes a stent body and a radiopaque structure. The stent body is formed by coupling at least one closed-loop net unit with each other. The radiopaque structure includes at least a main radiopaque wire, and the main radiopaque wire is wound around at least part of the stent body along the struts. The main radiopaque wire forms a radiopaque net unit in a path in which the struts are wound around the stent body. The described arrangement allows the structure and/or position of the stent body to be indicated by the radiopaque net unit, thereby facilitating a surgeon determining the position of the stent body, providing more information for the surgeon to determine the position of the stent, and further improving visibility of the stent body.
(2) Further, the first closed-loop net unit and the second closed-loop net unit of the stent body are each enclosed by struts, and the enclosed area of the first closed-loop net unit is larger than the enclosed area of the second closed-loop net unit, so that it can improve the efficiency in embedding into thrombus and improve the efficiency of thrombectomy.
(3) Further, the proximal radiopaque member and the distal radiopaque member are respectively provided on the stent body. Therefore, the stent body can be fully indicated to the surgeon by the proximal radiopaque member, the distal radiopaque member and the main radiopaque member at any time of the operation.

### BRIEF DESCRIPTION OF THE DRAWINGS

Fig. 1 shows a schematic diagram showing the flattened configuration of the stent and the thrombectomy system including the stent according to the present invention.
Fig. 2 is a structural schematic diagram of the stent in the thrombectomy system according to an embodiment of the present invention.
Fig. 3 is a structural schematic diagram of the closed-loop net unit of the stent in the thrombectomy system according to an embodiment of the present invention.
Fig. 4 is a schematic diagram showing the relation between the area of the radiopaque net unit and the area of the closed-loop net unit of the stent in the thrombectomy system according to an embodiment of the present invention.
Fig. 5 is a schematic diagram of a first winding path for the main radiopaque member of the stent in the thrombectomy system according to a first embodiment of the present invention.
Fig. 6 is a schematic diagram of a second winding path for the main radiopaque member of the stent in the thrombectomy system according to a first embodiment of the present invention.
Fig. 7 is a schematic diagram of a third winding path for the main radiopaque member of the stent in the thrombectomy system according to a first embodiment of the present invention.
Fig. 8 is a schematic diagram of a fourth winding path for the main radiopaque member of the stent in the thrombectomy system according to a first embodiment of the present invention.
Fig. 9 is a schematic diagram of a winding path combining the first winding path with the second winding path for the main radiopaque member of the stent in the thrombectomy system according to a first embodiment of the present invention.
Fig. 10 is a schematic diagram of a first winding path for the main radiopaque member of the stent in the thrombectomy system according to a second embodiment of the present invention.
Fig. 11 is a schematic diagram of a second winding path for the main radiopaque member of the stent in the thrombectomy system according to a second embodiment of the present invention.
Fig. 12 is a schematic diagram of a third winding path for the main radiopaque member of the stent in the thrombectomy system according to a second embodiment of the present invention.
Fig. 13 is a schematic diagram of a fourth winding path for the main radiopaque member of the stent in the thrombectomy system according to a second embodiment of the present invention.
Fig. 14 is a schematic diagram of a fifth winding path for the main radiopaque member of the stent in the thrombectomy system according to a second embodiment of the present invention.
Fig. 15 is a schematic diagram of a sixth winding path for the main radiopaque member of the stent in the thrombectomy system according to a second embodiment of the present invention.
Fig. 16 is a schematic diagram of a winding path combining the first winding path with the second winding path for the main radiopaque member of the stent in the thrombectomy system according to a second embodiment of the present invention.
Fig. 17 is a schematic diagram of a winding path combining the fifth winding path with the sixth winding path for the main radiopaque member of the stent in the thrombectomy system according to a second embodiment of the present invention.
Fig. 18 is a schematic diagram of a first winding path for the main radiopaque member of the stent in the thrombectomy system according to a third embodiment of the present invention.
Fig. 19 is a schematic diagram of a second winding path for the main radiopaque member of the stent in the thrombectomy system according to a third embodiment of the present invention.
Fig. 20 is a schematic diagram of a third winding path for the main radiopaque member of the stent in the thrombectomy system according to a third embodiment of the present invention.
Fig. 21 is a schematic diagram of a fourth winding path for the main radiopaque member of the stent in the thrombectomy system according to a third embodiment of the present invention.
Fig. 22 is a schematic diagram showing a first exemplary structure of the distal radiopaque member of the stent in the thrombectomy system according to an embodiment of the present invention.
Fig. 23 is a right-side cross-sectional view of the distal radiopaque member of the stent in the thrombectomy system according to an embodiment of the present invention.
Fig. 24 is a front cross-sectional view of the distal radiopaque member of the stent in the thrombectomy system according to an embodiment of the present invention.
Fig. 25 is a schematic diagram showing a second exemplary structure of the distal radiopaque member of the stent in the thrombectomy system according to an embodiment of the present invention.
Fig. 26 is an isometric view showing the second exemplary structure of the distal radiopaque member of the stent in the thrombectomy system according to an embodiment of the present invention.
Fig. 27 is a top view of the second exemplary structure of the distal radiopaque member of the stent in the thrombectomy system according to an embodiment of the present invention.
Fig. 28 is a front view of the second exemplary structure of the distal radiopaque member of the stent in the thrombectomy system according to an embodiment of the present invention.
Fig. 29 is a bottom view of the second exemplary structure of the distal radiopaque member of the stent in the thrombectomy system according to an embodiment of the present invention.
Fig. 30 is a schematic diagram showing a third exemplary structure of the distal radiopaque member of the stent in the thrombectomy system according to an embodiment of the present invention.
Fig. 31 is a structural schematic diagram of the proximal radiopaque member of the stent in the thrombectomy system according to an embodiment of the present invention.

### List of reference numerals:

1, Distal Radiopaque Member; 101, First Distal Radiopaque Wire; 102, Second Distal Radiopaque Wire; 103, Third Distal Radiopaque Wire; 2, Main Radiopaque Member; 201, First Main Radiopaque Wire; 202, Second Main Radiopaque Wire; 203, Third Main Radiopaque Wire; 3, Proximal Radiopaque Member; 301, Proximal Radiopaque Wire; 4, Stent Body; 41, Distal Portion; 411, Arcuate Portion; 42, Middle Portion; 43, Proximal Portion; 430, First Slope Strut; 431, Second Slope Strut; 400, First Closed-loop Net Unit; 4001, First Long Strut; 4002, First Short Strut; 4003, Second Long Strut; 4004, Second Short Strut; 401, Second Closed-loop Net Unit; 4011, Third Short Strut; 5. Push Rod.

### DETAILED DESCRIPTION

In order to make the objects, technical solutions and advantages of the present invention more clear, the stent and the thrombectomy system including the stent according to the present application will be further described in detail below in conjunction with the drawings and specific embodiments. Advantages and features of the present invention will be apparent from the description below. It is noted that the figures are provided in a very simplified form not necessarily presented to scale, with their only intention to facilitate convenience and clarity in explaining the disclosed embodiments. In order to make the objects, features and advantages of the present invention more apparent, please refer to the accompanying drawings. It should be noted that the structures, proportions, sizes, etc. shown in the drawings attached to this specification are only used to coordinate with the content disclosed in the specification for the understanding and reading of those familiar with this technology, and are not used to limit the implementation of the present invention. It has no technical substantive significance. Any structural modifications, changes in proportions, or adjustments in size should still fall within the scope of the present invention without affecting the efficacy and purpose of the present invention.

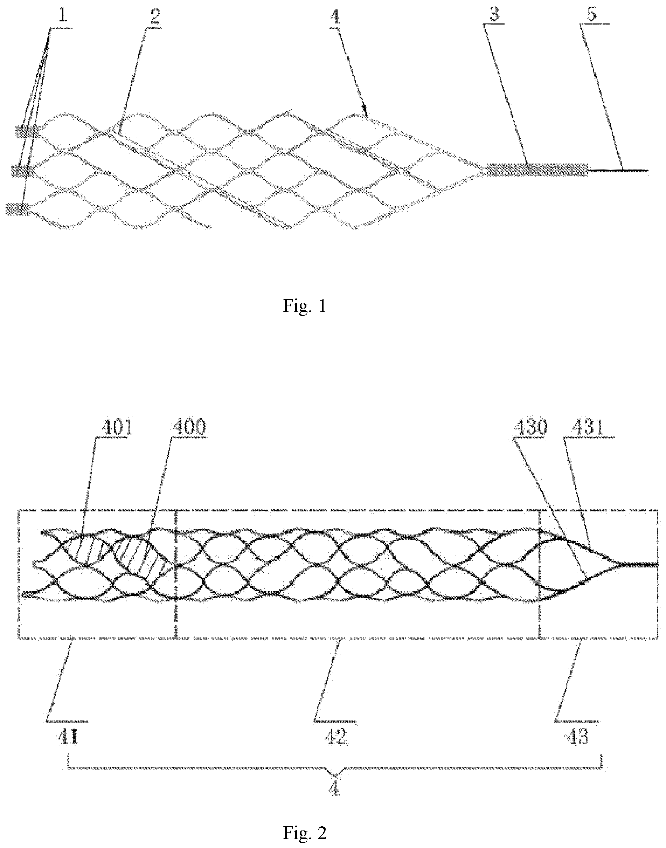

As used in this specification and the appended claims, the singular forms "a", "an", and "the" include plural referents unless the content clearly dictates otherwise. The term "proximal" usually refers to the side closer to the operator, and the term "distal" usually refers to the side closer to the patient's diseased part. Taking Fig. 1 as an example, the left side in Fig. 1 is the distal side, and the right side in Fig. 1 is the proximal side.

### Embodiment 1

The specific structure of the stent is described below:
Please refer to Figs. 1 and 2, the stent includes a stent body 4 and a radiopaque structure.

The stent body 4 has a cylindrical structure formed by at least a closed-loop net unit coupled with each other. The stent body 4 is not limited to having the cylindrical structure. In other embodiments of the present invention, it may have a spherical structure, a wedge-shaped structure, a spindle-shaped structure, or any one or a combination of other irregular structures. The closed-loop net unit is enclosed by struts.

The radiopaque structure includes a main radiopaque member 2. The main radiopaque member 2 includes at least one main radiopaque wire. The main radiopaque wire is wound around at least part of the stent body 4 along the struts. Optionally, the at least one main radiopaque wire is spirally wound around the struts. The at least one main radiopaque wire is arranged from the proximal end to the distal end of the stent body 4 in the stent of the first embodiment.

In other embodiments of the present invention, the at least one main radiopaque wire may be arranged from the distal end to the proximal end of the stent body 4, and the present invention is not limited thereto.

The specific structure of the stent body 4 is described below.

Please continue to refer to Figs. 1 and 2, the stent body 4 can self-expand in the circumferential direction along the longitudinal axis. The stent body 4 includes a distal portion 41, a middle portion 42 and a proximal portion 43 connected in sequence. The above-mentioned closed-loop net unit includes at least a first closed-loop net unit 400 and at least a second closed-loop net unit 401.

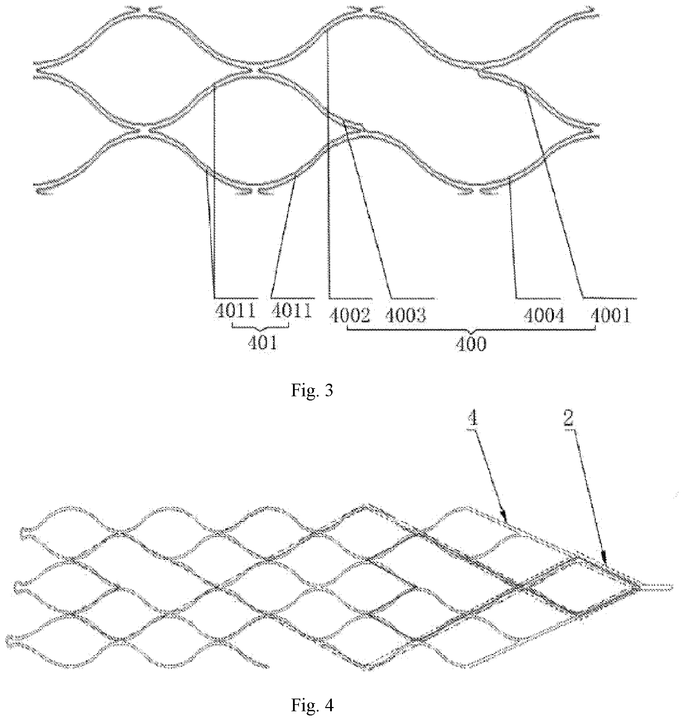

Specifically, the above-mentioned first closed-loop net unit 400 has a large mesh cell, which allows the stent body 4 to be effectively embedded into the thrombus when it is expanded among the thrombus, leading to a difficulty in fragmenting or cutting the thrombus into small pieces. As a result, it is easy to remove the thrombus completely. Please refer to Fig. 3. The first closed-loop net unit 400 may be an ordinary closed structure with a mesh cell which may be shaped as a circle, a rhombus, a hexagon, etc. In specific implementation, in order to further increase the flexibility and wall compliance of the overall spiral structure of the stent body 4, the first closed-loop net unit 400 includes one first long strut 4001 and one second long strut 4003 arranged in parallel with each other, and one first short strut 4002 and one second short strut 4004 arranged in parallel with each other. A quasi-quadrilateral structure is formed by enclosing the first long strut 4001, the second long strut 4003, the first short strut 4002 and the second short strut 4004.

Further the second closed-loop net unit 401 has a small mesh cell, which allows the stent body 4 to generate a large radial support force when catching the thrombus during the release process. As a result, it is beneficial to pull the thrombus when the stent body 4 is withdrawn, and it is not easy for the thrombus to fall off during the withdrawal process. Please refer to Figs. 2 and 3, the second closed-loop net unit 401 has also a closed structure with a mesh cell which may also be shaped as a circle, a rhombus, a hexagon, etc. In specific implementation, the second closed-loop net unit 401 has a quasi-quadrilateral structure enclosed by a plurality of third short struts 4011. If the second closed-loop net unit 401 is adjacent to the first closed-loop net unit 400 as shown Figs. 1 and 2, the second closed-loop net unit 401 will share one strut or a part thereof with the first closed-loop net unit 400. The shared strut may be any one of the first short strut 4002 and the second short strut 4004 of the first closed-loop net unit 400, or may be a portion of any one of the first long strut 4001 and the second long strut 4003 of the first closed-loop net unit 400.

Further, the area enclosed by the first closed-loop net unit 400 is larger than the area enclosed by the second closed-loop net unit 401. The above-mentioned arrangement of the first closed-loop net unit 400 with a large mesh cell and the second closed-loop net unit 401 with a small mesh cell can improve the efficiency in embedding into thrombus and improve the efficiency of thrombectomy.

Further, in the stent of the first embodiment, the area enclosed by the first closed-loop net unit 400 is 2 to 5 times the area enclosed by the second closed-loop net unit 401. Optionally, the area enclosed by the first closed-loop net unit 400 is 2 to 4 times the area enclosed by the second closed-loop net unit 401.

Further, in the stent of the first embodiment, the number of the first closed-loop net units 400 is 1/20 to 1/3 of the number of the second closed-loop net units 401. Optionally, the number of the first closed-loop net units 400 is 1/14 to 1/4 of the number of the second closed-loop net units 401.

Further, please refer to Fig. 2, the proximal portion 43 of the stent body 4 also includes a slope strut arranged at a certain angle with the axis of the stent body 4. In the stent according to the first embodiment of the present invention, the first slope strut 430 and the second slope strut 431 each serves as the above-mentioned slope strut.

The specific structure of the main radiopaque member 2 is described below.

Please refer to Fig. 1, the main radiopaque member 2 includes a main radiopaque wire which forms a radiopaque net unit in the path the struts wound around the stent body 4. The radiopaque net unit is configured to indicate the structure and/or the position of the stent.

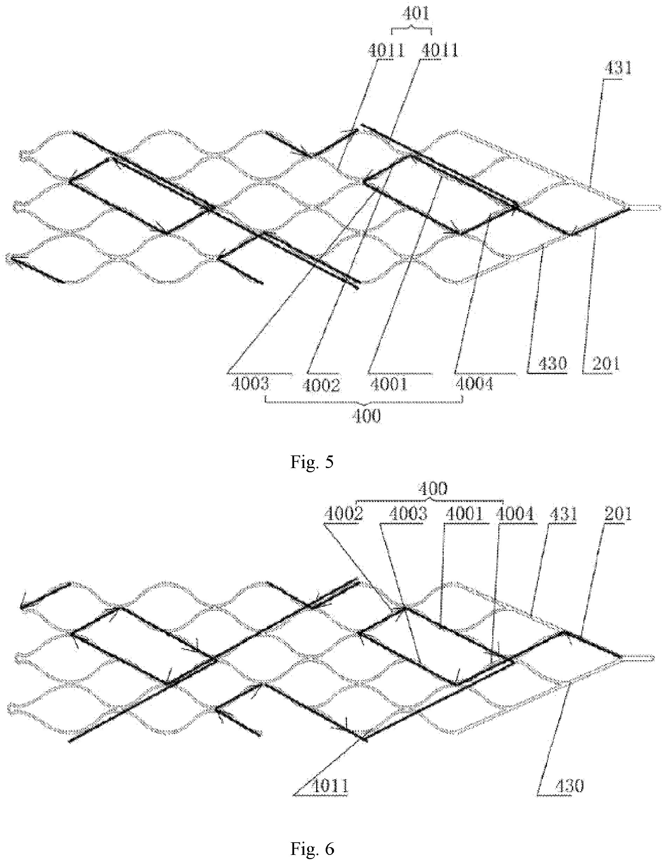

Further, the radiopaque net unit of the stent in the first embodiment has a closed geometric shape. The area of the radiopaque net unit is same as the area of the closed-loop net unit of the stent body 4 at the same position of the radiopaque net unit. The area of the radiopaque net unit refers to the area enclosed by the main radiopaque wires, and the area of the closed-loop net unit refers to the area enclosed by the struts. For example, please refer to Fig. 5, a single area enclosed by the first main radiopaque wire 201 is the area of the radiopaque net unit. The area of the radiopaque net unit is same as the area of the closed-loop net unit of the stent body 4 at this position enclosed by the struts (i.e., the first long strut 4001, the first short strut 4002, the second long strut 4003, and the second short strut 4004).

Further, the radiopaque net unit is configured to indicate a special position of the stent, and the special position is at any one or more of 1/3, 1/2 or 2/3 of the total length of the stent. The special position indicated by the radiopaque net unit provides the surgeon with the information about the position and posture of the stent, making it easier for the surgeon to determine the operation.

Of course, in other embodiments of the present invention, the area of the radiopaque net unit may also be smaller than the area of the closed-loop net unit of the stent body 4 at the position of the radiopaque net unit. Alternatively, please refer to Fig. 4, the area of the radiopaque net unit is the sum of the areas of several closed-loop net units of the stent body 4 at the position of the radiopaque net unit.

Please refer to Fig. 5, the main radiopaque member 2 is at least one first main radiopaque wire 201 wound onto all the struts of at least one first closed-loop net unit 400 and onto a part of the struts of at least one second closed-loop net unit 401.

Further, please continue to refer to Fig. 5, the winding direction of the first main radiopaque wire 201 is from the proximal end of the stent body 4 to the distal end of the stent body 4. Specifically, the first main radiopaque wire 201 is wound in the direction of the arrow from the proximal end of the first slope strut 430, and is then wound onto three first closed-loop net units 400 in sequence upon a direction change occurs at the first bifurcation of the first slope strut 430, so as to reach the distal end of the stent body 4. Specifically, the first main radiopaque wire 201 is wound anticlockwise onto the first closed-loop net unit 400 along the first long strut 4001, the first short strut 4002, the second long strut 4003 and the second short strut 4004 in sequence. The first main radiopaque wire 201 is wound twice at the first long strut 4001 and enters the next first closed-loop net unit 400 along the winding path. The first main radiopaque wire 201 forms a radiopaque net unit by winding around all the struts of the first closed loop net unit 400, so that the first closed loop net unit 400 can be radiopaque, which facilitates indicating the position of the first closed loop net unit 400 and facilitate the surgeon's operation.

Further, please continue to refer to Fig. 5, when a next first closed-loop net unit 400 is to be wound by the first main radiopaque wire 201 after the winding for the current first closed-loop net unit 400 is complete, the first main radiopaque wire 201 needs to be wound around one third short strut 4011 of the second closed-loop net unit 401 so as to enter the winding path of the next first closed-loop net unit 400.

In other embodiments of the present invention, the winding path of the first main radiopaque wire 201 can be changed as follows.

Please refer to Fig. 6, it shows a second exemplary winding path of the first main radiopaque wire 201 of the main radiopaque member 2. The first main radiopaque wire 201 is still wound from the proximal end of the stent body 4 to the distal end of the stent body 4. Specifically, the first main radiopaque wire 201 is wound in the direction of the arrow from the proximal end of the second slope strut 431, and is then wound onto three first closed-loop net units 400 in sequence upon a direction change occurs at the first bifurcation of the second slope strut 431, so as to reach the distal end of the stent body 4. Specifically, the first main radiopaque wire 201 is wound clockwise onto all the struts of the first closed-loop net unit 400 along the second short strut 4004, the second long strut 4003, the first short strut 4002 and first long strut 4001 in sequence. The first main radiopaque wire 201 is wound twice at the second short strut 4004.

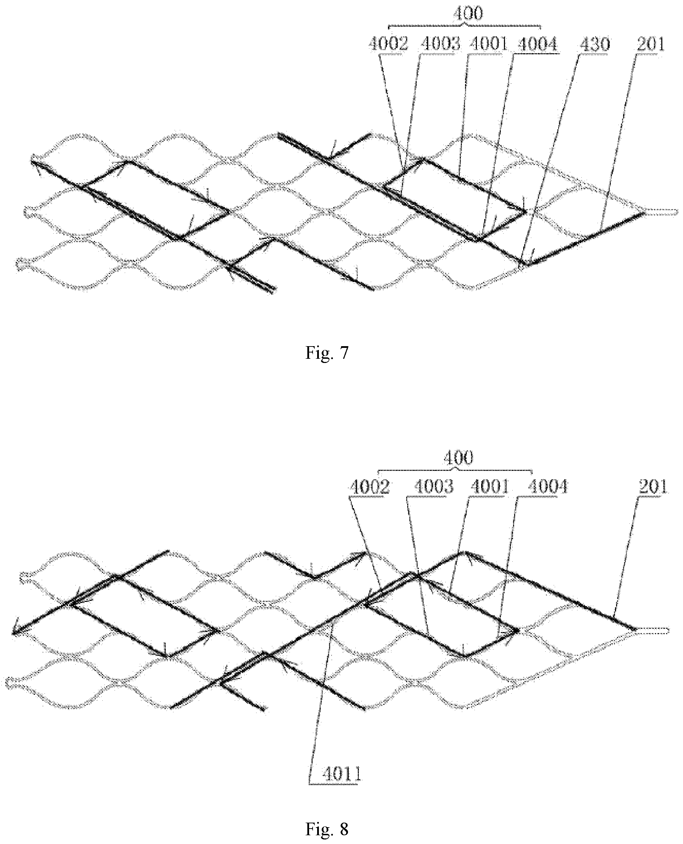

Please refer to Fig. 7, it shows a third exemplary winding path of the first main radiopaque wire 201 of the main radiopaque member 2. The first main radiopaque wire 201 is still wound from the proximal end of the stent body 4 to the distal end of the stent body 4. Specifically, the first main radiopaque wire 201 is wound in the direction of the arrow from the proximal end of the first slope strut 430 to the middle portion thereof, and is then wound onto three first closed-loop net units 400 in sequence upon a direction change occurs at the second bifurcation of the first slope strut 430, so as to reach the distal end of the stent body 4. Specifically, the first main radiopaque wire 201 is wound clockwise onto all the struts of the first closed-loop net unit 400 along the second long strut 4003, the first short strut 4002, the first long strut 4001 and the second short strut 4004 in sequence. The first main radiopaque wire 201 is wound twice at the second long strut 4003.

Please refer to Fig. 8, it shows a fourth exemplary winding path of the first main radiopaque wire 201 of the main radiopaque member 2. The first main radiopaque wire 201 is still wound from the proximal end of the stent body 4 to the distal end of the stent body 4. Specifically, the first main radiopaque wire 201 is wound in the direction of the arrow from the proximal end to the distal end of the second slope strut 431, and is then wound onto three first closed-loop net units 400 in sequence upon a direction change occurs at the third bifurcation of the second slope strut 431, so as to reach the distal end of the stent body 4. Specifically, the first main radiopaque wire 201 is wound anticlockwise onto all the struts of the first closed-loop net unit 400 along the first short strut 4002, the second long strut 4003, the second short strut 4004 and the first long strut 4001 in sequence. The first main radiopaque wire 201 is wound twice at the first short strut 4002.

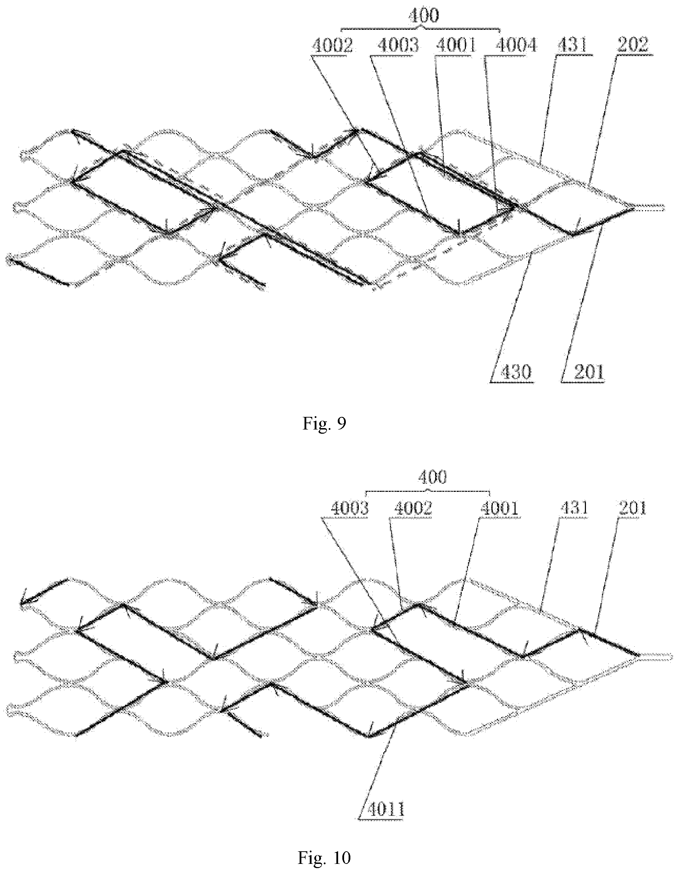

Please refer to Fig. 9, in other embodiments of the present invention, the main radiopaque member 2 includes more than one main radiopaque wire. In Fig. 9, the main radiopaque member 2 includes a first main radiopaque wire 201 and a second main radiopaque wire 202. The first main radiopaque wire 201 is wound in the first exemplary winding path of the first main radiopaque wire 201 shown in Fig. 5, and the second main radiopaque wire 202 is wound in the second exemplary winding path shown in Fig. 6. The combination of the first main radiopaque wire 201 and the second main radiopaque wire 202 enables the position of the first closed-loop net unit 400 to be more clearly indicated, thereby facilitating the surgeon's operation.

In other embodiments of the present invention, the winding path of the main radiopaque member 2 may also be any combination of the winding paths shown in Figs. 5 to 8, as long as it can clearly indicate the position of the first closed-loop net unit 400 so as to facilitate the surgeon's operation.

Further, the stent according to the first embodiment of the present invention may also include a distal radiopaque member 1 and/or a proximal radiopaque member 3. Please refer to Fig. 1, the distal radiopaque member 1 is fixed to the distal end of the stent body 4. The proximal radiopaque member 3 is fixed to the proximal end of the stent body 4.

The specific structure of the distal radiopaque member 1 is described below.

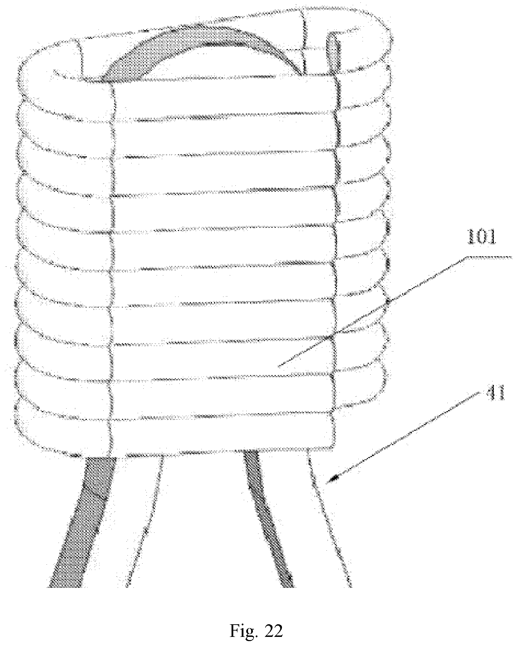

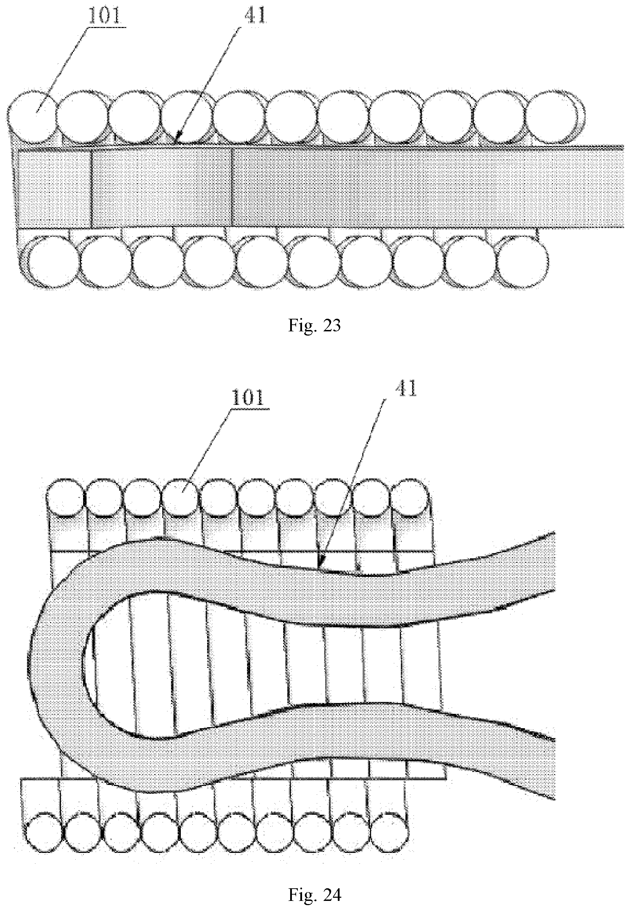

Please refer to Figs. 22 to 24, in the stent according to the first embodiment of the present invention, the distal radiopaque member 1 is sleeved on the distal strut of the stent body 4. The distal radiopaque member 1 is wound onto the distal strut of the stent body 4 to form at least one layer of wrapping structure thereon. Specifically, in the first embodiment of the present invention, the first distal radiopaque wire 101 is wound several turns around the distal end of the stent body 4 from proximal to distal (or from distal to proximal) to form one layer of wrapping structure.

Further, please refer to Figs. 1, 22 to 24, the proximal end of the distal radiopaque member 1 is integrally or detachably connected to the distal end of the main radiopaque member 2. When the proximal end of the distal radiopaque member 1 is integrally connected to the main radiopaque member 2, they are formed by one radiopaque wire. When the proximal end of the distal radiopaque member 1 is detachably connected to the distal end of the main radiopaque member 2, they may be fixedly connected by bonding or winding. Of course, the proximal end of the distal radiopaque member 1 may be fixedly connected to the distal end of the main radiopaque member 2 by welding. In some embodiments, the distal radiopaque member 1 and the main radiopaque member 2 may be independent of each other and not connected.

In other embodiments of the present invention, the first distal radiopaque wire 101 may be wound several turns onto the distal strut of the stent body 4 from the proximal end to the distal end of the stent body 4 (or from the distal end to the proximal end of the stent body 4) to form a layer of wrapping structure, and then wound several turns to form a second layer of wrapping structure or further layers of wrapping structure.

Further, in other embodiments of the present invention, the distal radiopaque member 1 may be fixed to the distal end of the stent body 4 by bonding, welding, etc.

The specific structure of the proximal radiopaque member 3 is described below.

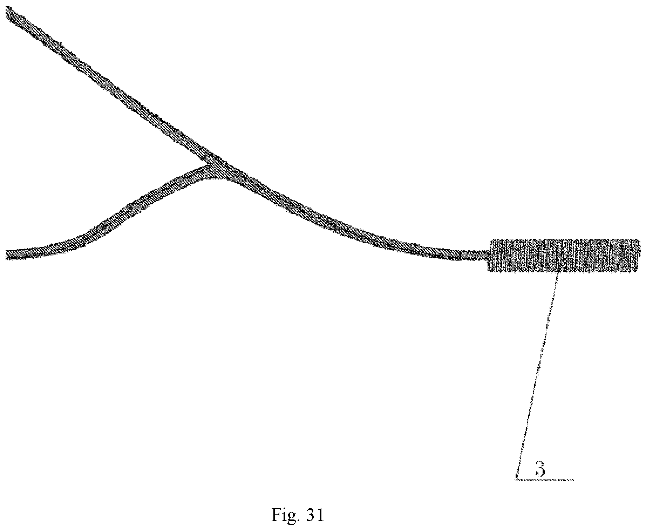

Please refer to Figs. 1 and 31, the proximal radiopaque member 3 is connected to the proximal end of the stent body 4. The proximal radiopaque member 3 may be fixed to the proximal end of the stent body 4 by sleeving, winding, welding or bonding. The distal end of the proximal radiopaque member 3 may be integrally or detachably connected to the proximal end of the main radiopaque member 2. In the stent according to the first embodiment of the present invention, the proximal radiopaque member 3 includes the proximal radiopaque wire 301 wound around the proximal end of the stent body 4.

Further, the number of radiopaque wires in each of the above-mentioned main radiopaque member 2, proximal radiopaque member 3 and distal radiopaque member 1 may be 1 to 8, and optionally is 1 to 6.

The present invention also provides a thrombectomy system, which includes the above-mentioned stent and a push wire 5 provided at the proximal end of the stent. The push wire 5 is arranged non-coaxially with the stent.

Further, the proximal radiopaque member 3 is arranged coaxially with the proximal end of the stent, and the distal radiopaque member 1 is arranged coaxially with the distal strut of the stent.

Further, the proximal radiopaque member 3, the main radiopaque member 2 and the distal radiopaque member 1 are made of radiopaque materials including any one or combination of tantalum, gold, platinum, platinum-iridium, and platinum-tungsten.

Further, the stent body 4 may be made of any one of nickel-titanium alloy, cobalt-based alloy or stainless steel by cutting a metal tube.

Further, the push wire 5 may be made of any one or more of stainless steel, cobalt-chromium-nickel alloy or nickel-titanium alloy.

In other embodiments of the present invention, the push wire and the stent in the thrombectomy system may be connected coaxially, and the proximal radiopaque member 3 may be arranged non-coaxially with the proximal end of the stent. The distal radiopaque member 1 may be arranged non-coaxially with the distal strut of the stent.

### Embodiment 2

The second embodiment of the present invention is basically same as the first embodiment, and the shared parts will not be described again. Only the differences will be described below.

The structure of the main radiopaque member 2 is described below.

In the stent of the second embodiment, the radiopaque net unit may have a semi-open geometric shape or a closed geometric shape formed from semi-open geometric shapes. Specifically, the main radiopaque member 2 is at least one first main radiopaque wire 201 that is wound around a part of the struts of the first closed-loop net unit 400 and wound around a part of the struts of the second closed-loop net unit 401.

Please refer to Fig. 10, it shows a first exemplary winding path of the first main radiopaque wire 201 of the main radiopaque member 2 according to the second embodiment of the present application. The first main radiopaque wire 201 is still wound from the proximal end of the stent body 4 to the distal end of the stent body 4. Specifically, the first main radiopaque wire 201 is wound in the direction of the arrow from the proximal end of the second slope strut 431, and is then wound onto three first closed-loop net units 400 in sequence upon a direction change occurs at the first bifurcation of the second slope strut 431, so as to reach the distal end of the stent body 4. Specifically, the first main radiopaque wire 201 is wound anticlockwise onto a part of the struts of the first closed-loop net unit 400 along the first long strut 4001, the first short strut 4002 and the second long strut 4003.

Please continue to refer to Fig. 10, when a next first closed-loop net unit 400 is to be wound by the first main radiopaque wire 201 after the winding for the current first closed-loop net unit 400 is complete, the first main radiopaque wire 201 needs to be wound onto two third short struts 4011 in sequence so as to be wound onto the next first closed-loop net unit 400.

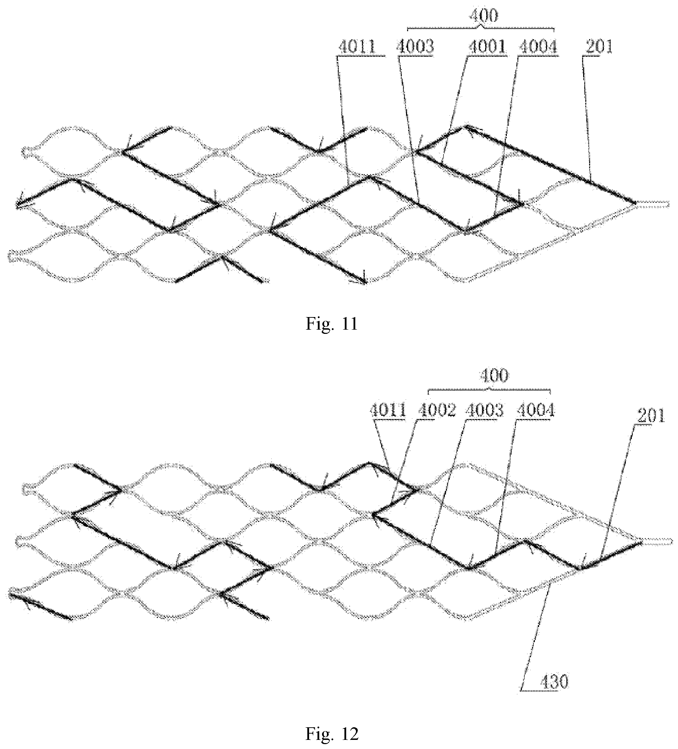

Please refer to Fig. 11, it shows a second exemplary winding path of the first main radiopaque wire 201 of the main radiopaque member 2 according to the second embodiment of the present application. The first main radiopaque wire 201 is still wound from the proximal end of the stent body 4 to the distal end of the stent body 4. Specifically, the first main radiopaque wire 201 is wound in the direction of the arrow from the proximal end to the distal end of the second slope strut 431, and is then wound onto three first closed-loop net units 400 in sequence upon a direction change occurs at the third bifurcation of the second slope strut 431, so as to reach the distal end of the stent body 4. Specifically, the first main radiopaque wire 201 is wound clockwise onto a part of the struts of the first closed-loop net unit 400 along the first long strut 4001, the second short strut 4004 and the second long strut 4003.

Please continue to refer to Fig. 11, when a next first closed-loop net unit 400 is to be wound by the first main radiopaque wire 201 after the winding for the current first closed-loop net unit 400 is complete, the first main radiopaque wire 201 needs to be wound onto two third short struts 4011 in sequence so as to be wound onto the next first closed-loop net unit 400. Before the first main radiopaque wire 201 is wound onto the first closed-loop net unit 400, the first main radiopaque wire 201 needs to be wound onto one of the third short struts 4011 of the second closed-loop net unit 401 from the distal end of the second slope strut 431.

Please refer to Fig. 12, it shows a third exemplary winding path of the first main radiopaque wire 201 of the main radiopaque member 2 according to the second embodiment of the present application. The first main radiopaque wire 201 is still wound from the proximal end of the stent body 4 to the distal end of the stent body 4. Specifically, the first main radiopaque wire 201 is wound in the direction of the arrow from the proximal end of the first slope strut 430, and is then wound onto three first closed-loop net units 400 in sequence upon a direction change occurs at the first bifurcation of the first slope strut 430, so as to reach the distal end of the stent body 4. Specifically, the first main radiopaque wire 201 is wound clockwise onto a part of the struts of the first closed-loop net unit 400 along the second short strut 4004, the second long strut 4003 and the first short strut 4002.

Please continue to refer to Fig. 12, when a next first closed-loop net unit 400 is to be wound by the first main radiopaque wire 201 after the winding for the current first closed-loop net unit 400 is complete, the first main radiopaque wire 201 needs to be wound onto one third short strut 4011 so as to be wound onto the next first closed-loop net unit 400.

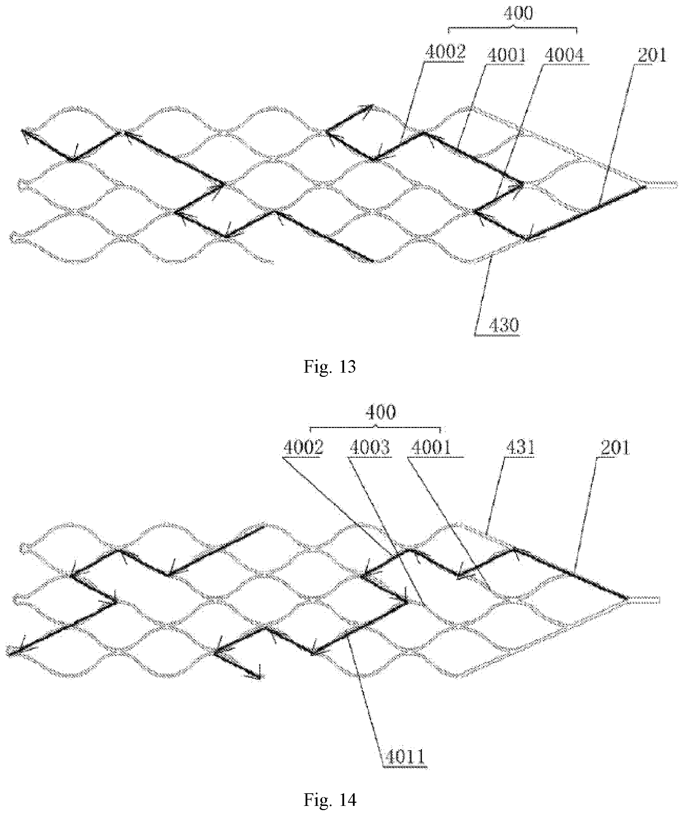

Please refer to Fig. 13, it shows a fourth exemplary winding path of the first main radiopaque wire 201 of the main radiopaque member 2 according to the second embodiment of the present application. The first main radiopaque wire 201 is still wound from the proximal end of the stent body 4 to the distal end of the stent body 4. Specifically, the first main radiopaque wire 201 is wound in the direction of the arrow from the proximal end of the first slope strut 430 to the middle portion thereof, and is then wound onto three first closed-loop net units 400 in sequence upon a direction change occurs at the second bifurcation of the first slope strut 430, so as to reach the distal end of the stent body 4. Specifically, the first main radiopaque wire 201 is wound anticlockwise onto a part of the struts of the first closed-loop net unit 400 along the second short strut 4004, the first long strut 4001 and the first short strut 4002.

Please refer to Fig. 14, it shows a fifth exemplary winding path of the first main radiopaque wire 201 of the main radiopaque member 2 according to the second embodiment of the present application. The first main radiopaque wire 201 is still wound from the proximal end of the stent body 4 to the distal end of the stent body 4. Specifically, the first main radiopaque wire 201 is wound in the direction of the arrow from the proximal end of the second slope strut 431 to the middle portion thereof, and is then wound onto three first closed-loop net units 400 in sequence upon a direction change occurs at the second bifurcation of the second slope strut 431, so as to reach the distal end of the stent body 4. Specifically, the first main radiopaque wire 201 is wound anticlockwise onto a part of the struts of the first closed-loop net unit 400 along a half of the first long strut 4001, the first short strut 4002 and a half of the second long strut 4003.

Please continue to refer to Fig. 14, when a next first closed-loop net unit 400 is to be wound by the first main radiopaque wire 201 after the winding for the current first closed-loop net unit 400 is complete, the first main radiopaque wire 201 needs to be wound onto two third short struts 4011 in sequence so as to be wound onto the next first closed-loop net unit 400. Before the first main radiopaque wire 201 is wound onto the first closed-loop net unit 400, the first main radiopaque wire 201 needs to be wound onto one of the third short struts 4011 of the second closed-loop net unit 401 from the middle portion of the second slope strut 431.

In other embodiments of the present invention, the length of the first long strut 4001 or the second long strut 4003 to be wound by the first main radiopaque wire 201 may be rather than a half thereof, for example, 1/3 length, 1/4 length of the first long strut 4001 or the second long strut 4003, as long as it allows the first main radiopaque wire 201 to be wound around the first long strut 4001 or the second long strut 4003.

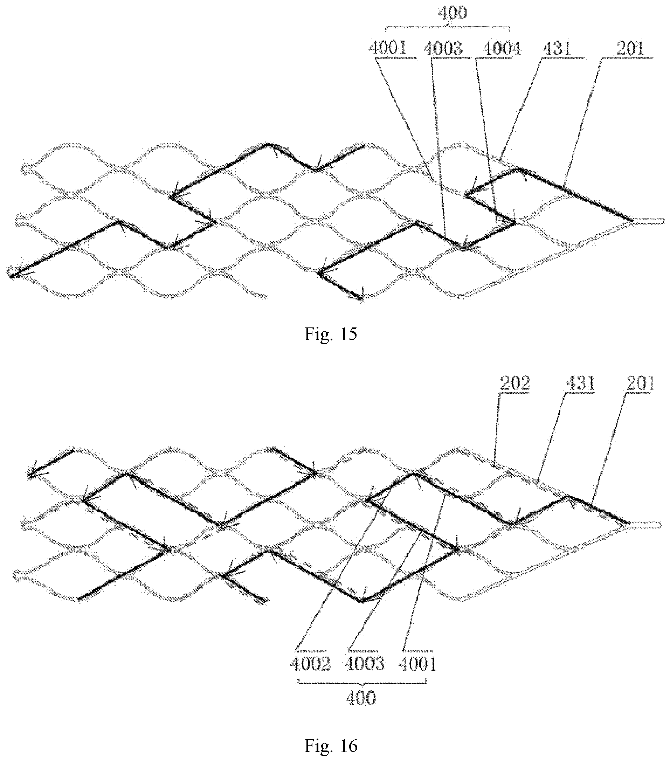

Please refer to Fig. 15, it shows a sixth exemplary winding path of the first main radiopaque wire 201 of the main radiopaque member 2 according to the second embodiment of the present application. The sixth exemplary winding path differs from the above five exemplary winding paths in that the first main radiopaque wire 201 is wound clockwise onto a part of the struts of the first closed-loop net unit 400 along a half of the first long strut 4001, the second short strut 4004 and a half of the second long strut 4003.

Please refer to Fig. 16, it is a schematic diagram showing the combination of the first exemplary winding path (please refer to Fig. 10) and the second exemplary winding path (please refer to Fig. 11) for the main radiopaque member according to the second embodiment of the present invention. In Fig. 16, the main radiopaque member 2 includes a first main radiopaque wire 201 and a second main radiopaque wire 202. The first main radiopaque wire 201 is wound onto the stent body 4 in the first exemplary winding path in Fig. 10, and the second main radiopaque wire 202 is wound onto the stent body 4 in the second exemplary winding path in Fig. 11. In Fig. 16, the first closed-loop net unit 400 is enclosed by the first main radiopaque wire 201 and the second main radiopaque wire 202. In a closed-loop net unit 400, the first long strut 4001 and the second long strut 4003 are respectively wound by the first main radiopaque wire 201 and the second main radiopaque wire 202. The combination of the first main radiopaque wire 201 and the second main radiopaque wire 202 can clearly indicate the position of the first closed-loop net unit 400, thereby facilitating the surgeon's operation.

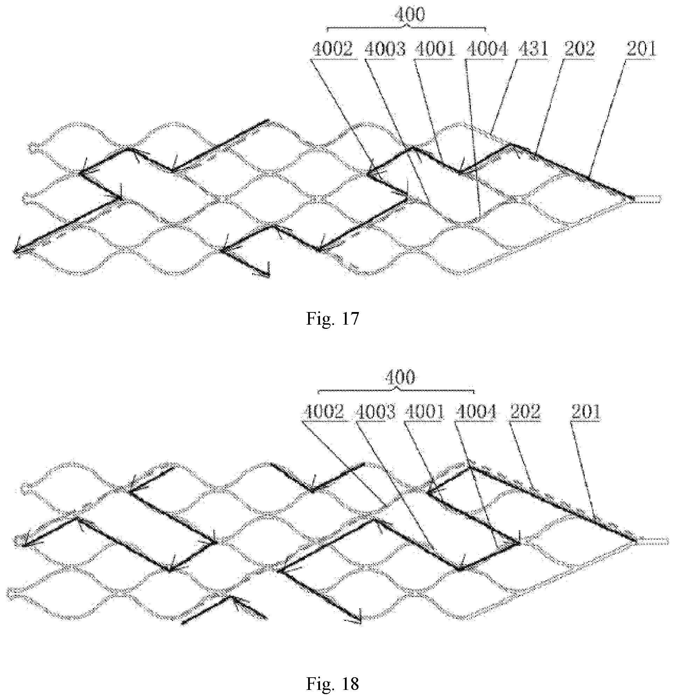

Please refer to Fig. 17, it is a schematic diagram showing the combination of the fifth exemplary winding path (please refer to Fig. 14) and the sixth exemplary winding path (please refer to Fig. 15) for the main radiopaque member according to the second embodiment of the present invention. In Fig. 17, the main radiopaque member 2 includes the first main radiopaque wire 201 and the second main radiopaque wire 202. The first closed loop net unit 400 is enclosed by the first main radiopaque wire 201 and the second main radiopaque wire 202, so that the position of a closed-loop net unit 400 can be clearly indicated to further facilitate the surgeon's operation.

The specific structure of the distal radiopaque member 1 is described below.

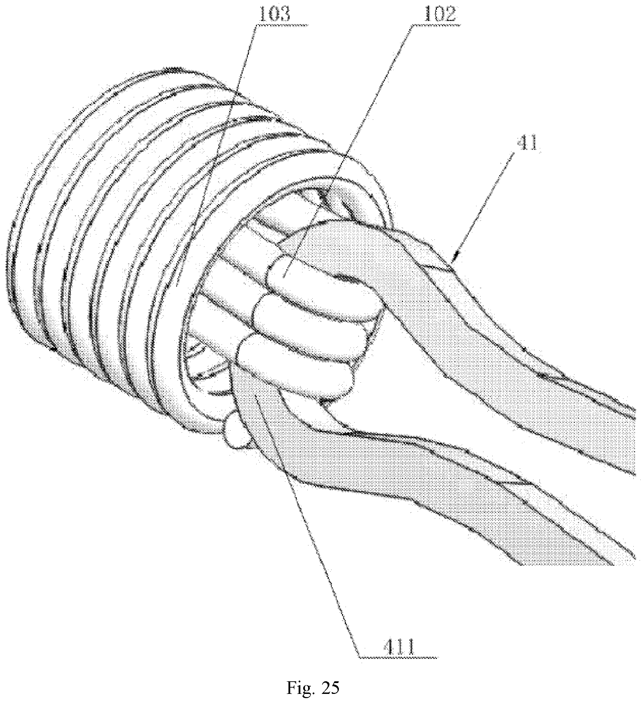

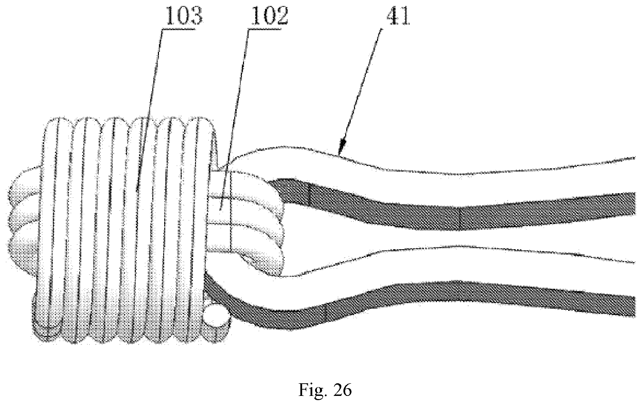

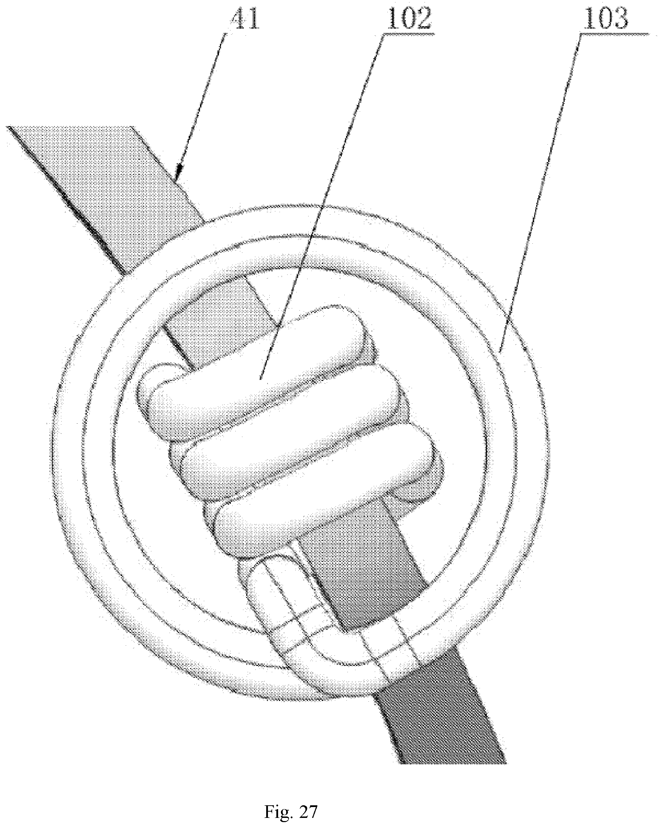

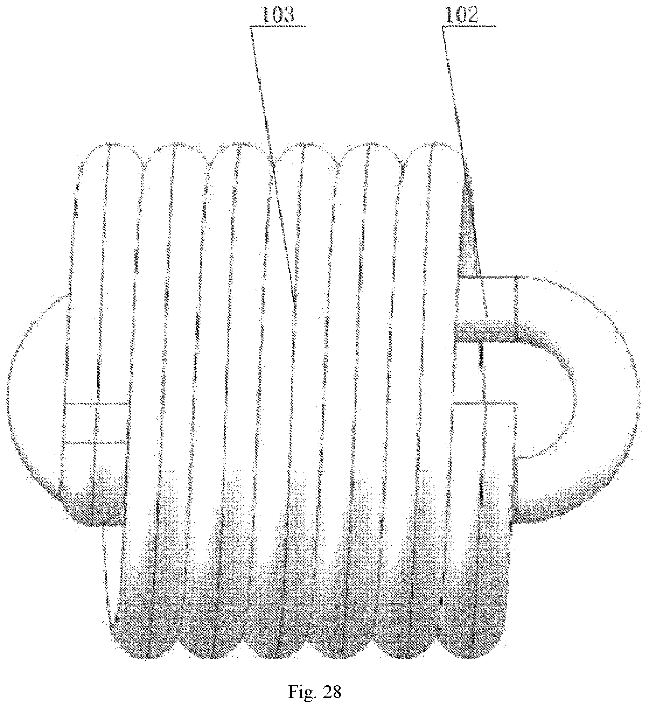

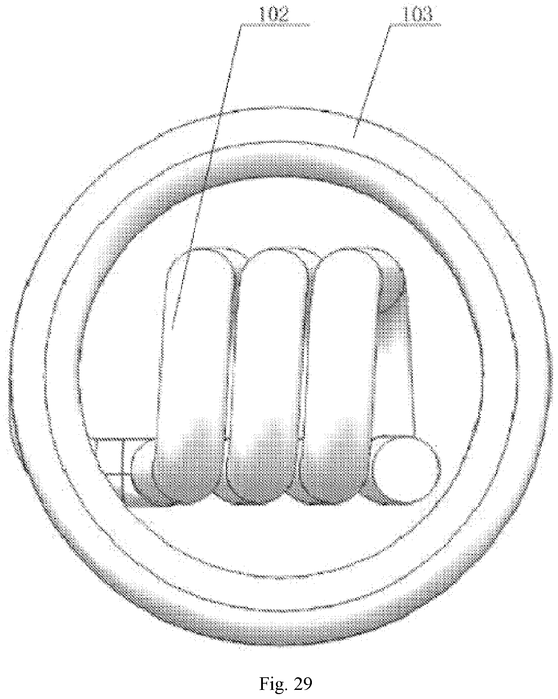

Please refer to Figs. 25 to 29, in the stent of the second embodiment of the present invention, the distal radiopaque member 1 is formed by a second distal radiopaque wire 102 and a third distal radiopaque wire 103 connected to the second distal radiopaque wire 102. The second distal radiopaque wire 102 is wound around the arcuate portion 411 of the distal portion 41 of the stent body 4 for several turns to form an axial ring. The proximal end of the third distal radiopaque wire 103 is connected to the outer side of the distal end of the second distal radiopaque wire 102, i.e., the tail portion of the axial ring. The third distal radiopaque wire 103 is wound around the outer side of the axial ring in the circumferential direction to form a circumferential ring.

### Embodiment 3

The stent in the third embodiment of the present invention is basically same as the first and second embodiments. The shared parts will not be described again, and only the differences will be described below.

The structure of main radiopaque member 2 is described below.

Please refer to Fig. 18, the main radiopaque member 2 includes a first main radiopaque wire 201 and a second main radiopaque wire 202. The first main radiopaque wire 201 is wound in the second exemplary winding path for the main radiopaque member in the second embodiment according to Fig. 11, and the second main radiopaque wire 202 is spirally wound from the proximal end of the stent body 4 to the distal end of the stent body 4. In this embodiment, the first short strut 4002 of the first closed loop net unit 400 is not wound by the first main radiopaque wire 201, but wound by the second main radiopaque wire 202, so that the first closed loop net unit 400 is enclosed and wound by the first main radiopaque wire 201 and the second main radiopaque wire 202. The combination of the first main radiopaque wire 201 and the second main radiopaque wire 202 enables the position of the first closed-loop net unit 400 to be clearly indicated, thereby facilitating the surgeon's operation.

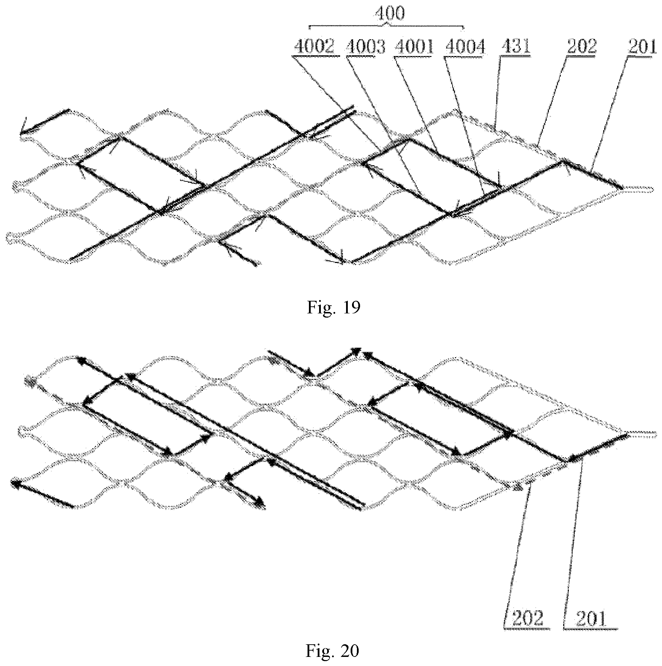

Further, please refer to Fig. 19, in other embodiments of the present invention, the main radiopaque member 2 includes the first main radiopaque wire 201 and the second main radiopaque wire 202. The first main radiopaque wire 201 is wound in the second exemplary winding path for the main radiopaque member 2 in the first embodiment according to Fig. 6, and the second main radiopaque wire 202 is spirally wound from the proximal end of the stent body 4 to the distal end of the stent body 4. Similarly, the first closed-loop net unit 400 is enclosed and wound by the first main radiopaque wire 201, and the second main radiopaque wire 202 is repeatedly wound onto the first short strut 4002. The combination of the first main radiopaque wire 201 and the second main radiopaque wire 202 enables the position of the first closed-loop net unit 400 to be clearly indicated, thereby facilitating the surgeon's operation.

Further, please refer to Fig. 20, the second main radiopaque wire 202 in other embodiments of the present invention is spirally wound from the proximal end of the stent body 4 to the distal end of the stent body 4, just like the second main radiopaque wire 202 in Fig. 19. The first main radiopaque wire 201 is wound in the first exemplary winding path for the main radiopaque member 2 in the first embodiment according to Fig. 5.

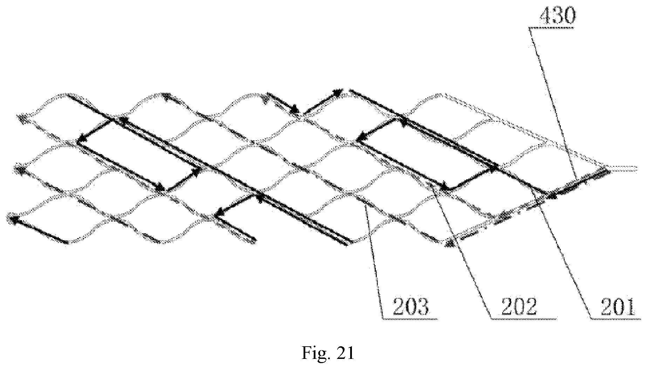

Further, please refer to Fig. 21, in other embodiments of the present invention, the main radiopaque member 2 may include three main radiopaque wires that are the first main radiopaque wire 201, the second main radiopaque wire 202 and the third main radiopaque wire 203. The first main radiopaque wire 201 is also wound in the first exemplary winding path for the main radiopaque member in the first embodiment according to Fig. 5. The second main radiopaque wire 202 is spirally wound from the proximal end to the distal end of the stent body 4 upon a direction change occurs at the middle portion (i.e., the second bifurcation) of the first slope strut 430. The third main radiopaque wire 203 is spirally wound from the proximal end to the distal end of the stent body 4 upon a direction change occurs at the distal end (i.e., the third bifurcation) of the first slope strut 430.

In other embodiments of the present invention, the main radiopaque member 2 may include 1 to 8 main radiopaque wires. In an embodiment, a plurality of main radiopaque wires are included, and the main radiopaque wires may be arranged according to any one of the first to third embodiments.

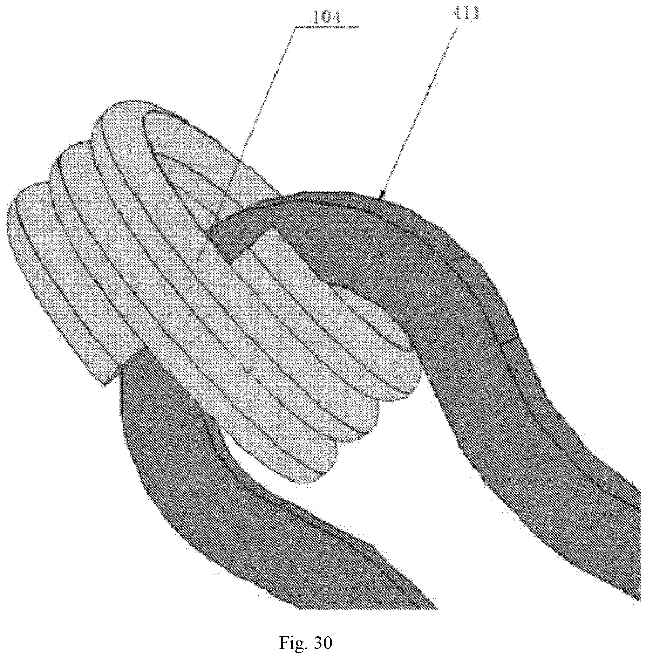

Please refer to Fig. 30, in the third embodiment of the present invention, the distal radiopaque member 1 is formed by the first distal radiopaque wire 101. The first distal radiopaque wire 101 is tightly wound around the arcuate portion 411 of the distal portion 41 of the stent body 4 for several turns to form an axial ring. The inner surface of the axial ring is in contact with the outer side of the arcuate portion 411.

The features of the above-described embodiments may be combined in any suitable manner. While not all possible combinations of these features are described for the sake of brevity, they are all considered within the scope of this specification as long as they there is no contradiction therein.

The above description provides a few embodiments of the present invention, and they are described above specifically and in detail, but they are not intended to be understood as limiting the scope of the present invention. It is noted that, although many variations and modifications can be made by those of ordinary skill in the art without departing from the spirit of the present invention, they all fall into the scope of protection of the present invention. Therefore, the scope of protection of the present invention should be determined by the appended claims.

## Claims

1. A stent, comprising a stent body and a radiopaque structure, the stent body formed by coupling at least one closed-loop net unit with each other, the closed-loop net unit each enclosed by struts, the radiopaque structure comprising a main radiopaque member having at least a main radiopaque wire wound around at least part of the stent body along the struts.

2. The stent according to claim 1, wherein the main radiopaque wire is spirally wound around the strut.

3. The stent according to claim 1, wherein the main radiopaque wire is arranged from a proximal end to a distal end of the stent body or from a distal end to a proximal end of the stent body.

4. The stent according to claim 1, wherein the main radiopaque wire forms a radiopaque net unit in a path in which the strut is wound around the stent body, and the radiopaque net unit is configured to indicate a structure and/or position of the stent.

5. The stent according to claim 4, wherein the radiopaque net unit has a closed geometric shape; or
wherein the radiopaque net unit has a semi-open geometric shape.

6. The stent according to claim 4, wherein an area of the radiopaque net unit is same as an area of the closed-loop net unit of the stent body at a position where the radiopaque net unit is; or
wherein an area of the radiopaque net unit is a sum of areas of a plurality of the at least one closed-loop net unit of the stent body at a position where the radiopaque net unit is.

7. The stent according to claim 1, wherein the closed-loop net unit each comprises at least a first closed-loop net unit and at least a second closed-loop net unit, and an area enclosed by the first closed-loop net unit is larger than an area enclosed by the second closed-loop net unit.

8. The stent according to claim 7, wherein the area enclosed by the first closed-loop net unit is 2 to 5 times the area enclosed by the second closed-loop net unit.

9. The stent according to claim 7, wherein a number of the first closed-loop net units is 1/20-1/3 of a number of the second closed-loop net units.

10. The stent according to claim 7, wherein the main radiopaque wire is wound onto at least one of the first closed-loop net units, so that the at least one of the first closed-loop net units of the stent body can be indicated.

11. The stent according to claim 10, wherein the main radiopaque wire is wound around all the struts of the at least one of the first closed-loop net units and wound around a part of the struts of at least one of the second closed-loop net units.

12. The stent according to claim 10, wherein a part of the struts of at least one of the first closed-loop net units is wound at least twice by the main radiopaque wire.

13. The stent according to claim 10, wherein the main radiopaque wire is wound around a part of the struts of the at least one of the first closed-loop net units and wound around a part of the struts of at least one of the second closed-loop net units.

14. The stent according to claim 10, wherein a number of the main radiopaque wires is at least two, and wherein one of the main radiopaque wires is wound around a part of the struts of the at least one of the first closed-loop net units and wound around a part of the struts of at least one of the second closed-loop net units, and another one of the main radiopaque wires is wound around a rest of the struts of the at least one of the first closed-loop net units and wound around a part of the struts of at least one of the second closed-loop net units.

15. The stent according to claim 1, wherein a number of the main radiopaque wires ranges from 1 to 8.

16. The stent according to any one of claims 1 to 15, wherein the radiopaque structure further comprises a distal radiopaque member connected to a distal end of the stent body.

17. The stent according to claim 16, wherein a proximal end of the distal radiopaque member is integrally or detachably connected to a distal end of the main radiopaque member.

18. The stent according to claim 16, wherein the distal radiopaque member is a first distal radiopaque wire that is wound around a distal strut of the stent body to form at least one layer of wrapping structure.

19. The stent according to claim 16, wherein the distal radiopaque member is a second distal radiopaque wire that is wound around a distal strut of the stent body to form an axial ring and further wound around an outer side of the axial ring to form a circumferential ring.

20. The stent according to claim 1, wherein the radiopaque structure further comprises a proximal radiopaque member connected to a proximal end of the stent body.

21. The stent according to claim 20, wherein a distal end of the proximal radiopaque member is integrally or detachably connected to a proximal end of the main radiopaque member.

22. A thrombectomy system, comprising the stent according to any one of claims 1 to 21 and a push rod provided at a proximal end of the stent, the push rod arranged coaxially or non-coaxially with the stent.
